# EUROPÄISCHE PATENTSCHRIFT

(11) **EP 1 092 036 B1**
(45) Veröffentlichungstag und Bekanntmachung des Hinweises auf die Patenterteilung: **24.08.2005**
(21) Anmeldenummer: 99924860.2
(22) Anmeldetag: 03.05.1999
(51) Int. Cl.: C12P 1/00, C12N 11/00, C12P 21/00, C12P 19/00, C12P 21/02, C07K 1/04, C07K 14/62, C12N 11/02, C12N 11/06

(54) **VERFAHREN ZUR KATALYSE VON KOMPLEXEN REAKTIONEN GROSSER MOLEKÜLE MITTELS AN EINEM POLYMEREN TRÄGER GEBUNDENER ENZYME**
METHOD FOR CATALYSING COMPLEX REACTIONS OF LARGE MOLECULES USING ENZYMES WHICH ARE BONDED TO A POLYMER SUPPORT
PROCEDE DE CATALYSE DE REACTIONS COMPLEXES DE GRANDES MOLECULES AU MOYEN D'ENZYMES LIEES A UN SUPPORT POLYMERE

(30) Priorität: 15.05.1998 DE 19821866
(43) Veröffentlichungstag der Anmeldung: 18.04.2001
(73) Patentinhaber: Aventis Pharma Deutschland GmbH, 65929 Frankfurt am Main (DE)
(72) Erfinder: BONGS, Jürgen, D-65189 Wiesbaden (DE); MEIWES, Johannes, D-65510 Idstein (DE)
(86) Internationale Anmeldenummer: PCT/EP1999/002973
(87) Internationale Veröffentlichungsnummer: WO 1999/060150

(56) Entgegenhaltungen:
- EP-A- 0 294 851
- US-A- 4 994 388
- CHEMICAL ABSTRACTS, vol. 120, no. 25, 20. Juni 1994 (1994-06-20) Columbus, Ohio, US; abstract no. 321404, HUWIG, ALEXANDER ET AL: "Laboratory procedures for producing 2-keto-D-glucose, 2-keto-D-xylose and 5-keto-D-fructose from D-glucose, D-xylose and L-sorbose with immobilized pyranose oxidase of Peniophora gigantea" XP002114237 & J. BIOTECHNOL. (1994), 32(3), 309-15 ,
- CHEMICAL ABSTRACTS, vol. 123, no. 19, 6. November 1995 (1995-11-06) Columbus, Ohio, US; abstract no. 250077, LORENZEN, P. CHR. ET AL: "Characterization of trypsin immobilized on oxirane-acrylic bead for obtaining phosphopeptides from casein" XP002114238 & Z. ERNAEHRUNGSWISS. (1995), 34(2), 118-30 ,
- CHEMICAL ABSTRACTS, vol. 122, no. 7, 13. Februar 1995 (1995-02-13) Columbus, Ohio, US; abstract no. 82031, ECKSTEIN, H. ET AL: "Immobilization of papain and trypsin on epoxy-polymers for enzyme-catalyzed peptide synthesis" XP002114239 & CHEM. PEPT. PROTEINS (1993), 5/6(PT. A), 211-16 ,
- JAKUBKE H.-D. ET AL.: "Grundprinzipien der Proteasekatalysierten Knüpfung der Peptidbindung." ANGEWANDTE CHEMIE, Bd. 97, Nr. 2, 1985, Seiten 79-87,
- KöNNECKE A. ET AL.: "Peptidsynthesen mit immobilisierten Enzymen II." MONATSHEFTE FüR CHEMIE, Bd. 114, Nr. 4, 1983, Seiten 433-444,
- JAKUBKE H.-D. ET AL.: "Application of immobilized proteases to peptide synthesis." ANALYTICAL CHEMISTRY SYMPOSIA SERIES, Bd. 9, 1982, Seiten 529-536,
- KöNNECKE A. ET AL.: "Ppetide synthesis by means of immobilized enzymes." MONATSHEFTE FüR CHEMIE, Bd. 112, Nr. 4, 1981, Seiten 469-481,

## Beschreibung

Die vorliegende Erfindung betrifft ein Verfahren zur Katalyse von komplexen Reaktionen großer Moleküle, nämlich Reaktionen mit unerwünschten Folge- oder Nebenreaktionen, mittels an einem polymeren Träger gebundener Enzyme und im besonderen ein Verfahren zur enzymatischen Gewinnung von Biomolekülen, insbesondere von biologisch aktiven Peptiden, beispielsweise von Insulinen oder deren Analoga, von Proteinen, Oligosacchariden oder von Polysacchariden, aus deren biologisch nicht aktiven Vorstufen bzw. Vorläufern, beispielsweise Präproinsulinen, mittels an einem polymeren Träger gebundener Enzyme.

Die Herstellung von Humaninsulin und seiner Derivate ist über unterschiedliche Ansätze in der Literatur beschrieben. Neben der chemischen Synthese, die auf Grund der Komplexität des Zielmoleküls unwirtschaftlich ist, existieren weiterhin ein semisynthetisches sowie ein gentechnisches Verfahren.

Beim semisynthetischen Ansatz kommt es zu einem durch Trypsin katalysierten Austausch der C-terminalen Aminosäure der B-Kette des Schweineinsulins, der zur Bildung des Humaninsulins führt (H.-D. Jakubke et al., Angew. Chem., 97 (1985) 79).

Der gentechnische Ansatz zur Herstellung von Humaninsulin verläuft über die Stufe des Präproinsulins und seiner Derivate, die im Rahmen der Aufarbeitung ebenfalls einer tryptischen Spaltung unterzogen werden (B.H. Frank et al., Peptides: synthesis, structure, function, (1981) 729-738; Jonasson et al., Eur. J. Biochem., 236 2 (1996) 656-661; Kemmler et al.,J. Biol. Chem., 246 (1971) 6786-6791).

Zur effizienteren Nutzung des Enzyms wurde für den semisynthetischen Ansatz ein Verfahren entwickelt, das den Einsatz von immobilisiertem Trypsin erlaubt (EP 0 294 851).

Für die Gewinnung von Insulinen oder dessen Analoga aus den entsprechenden, gentechnisch hergestellten Präproinsulinen ist bislang kein enzymatisches Verfahren bekannt, bei welchem das Enzym Trypsin immobilisiert vorliegt, so daß bei bekannten Verfahren einerseits für jeden neuen Reaktionsansatz neues Trypsin zugegeben werden muß und andererseits im Rahmen der Produktaufreinigung eine aufwendige Abreicherung des Enzyms notwendig ist.

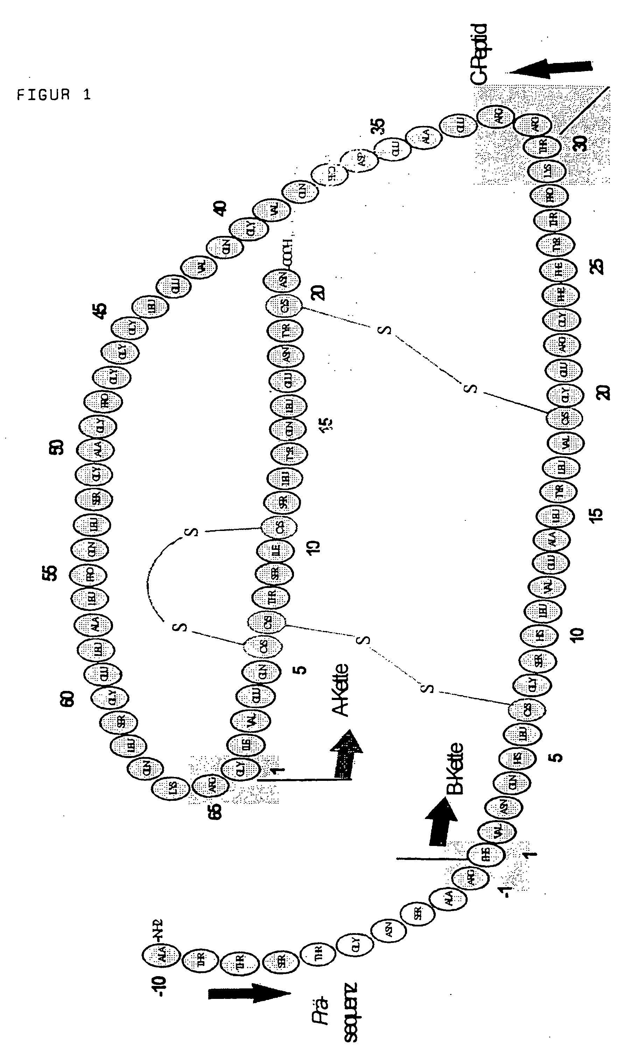

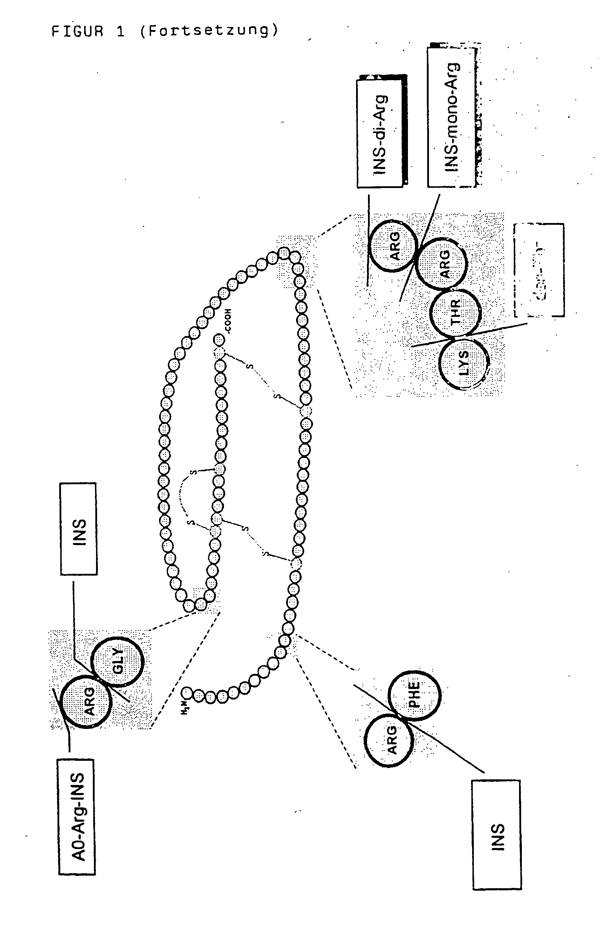

Die tryptische Spaltung von Präproinsulin (PPI) ist eine komplexe, enzymkatalysierte Reaktion mit zahlreichen unerwünschten Folge- und Nebenreaktionen. Wie in Fig. 1 gezeigt, entstehen bei der tryptischen Spaltung von PPI auf Grund der zahlreichen reaktiven Stellen eine Velzahl von Reaktionsprodukten, von denen die Verbindungen Arg(B31),Arg(B32)-Insulin ("di-Arg") und Arg(B31)-Insulin ("mono-Arg") als die eigentlichen Wertsstoffe für die weitere Aufarbeitung anzusehen sind. Somit ist eine Abspaltung sowohl der Präsequenz als auch der "mittigen" (der im Präproinsulin zwischen der Sequenz der A-Kette und der Sequenz der B-Kette des Insulins angeordneten) C-Kette notwendig. Kommt es an anderen Stellen des PPI's zu Spaltreaktionen, so entstehen unerwünschte Nebenprodukte wie z. B. das Des(B30)-Insulin ("des-Thr").

Während die Spaltung mit nativem Trypsin zur vorwiegenden Bildung der beiden Wertstoffe führt, Arg(B31),Arg(B32)-Insulin ("di-Arg") und Arg(B31)-Insulin ("mono-Arg"), liefert der Einsatz von Trypsin-Immobilisaten auf der Grundlage konventioneller Träger wie z. B. Eupergit® C250L, Eupergit® C, Deloxan® ein unbefriedigendes Reaktionsmuster. Die beiden Wertstoffe di- und mono-Arg werden dabei in nur geringen Anteilen gebildet, während in erster Linie die unerwünschten Folge- bzw. Nebenprodukte (vornehmlich des-Thr) entstehen.

Huwig et al. (Chemical Abstracts, vol. 120, no. 25, 20. Juni 1994) beschreiben ein Laborverfahren zur Herstellung von 2-keto-D-Glucose und anderer Zucker mit immobilisierter Pyranoseoxidase von Peniophora gigantea. Lorenzen und Schlumme (Chemical Abstracts, vol. 123, no. 19, 6. November 1995) beschreiben die Charakterisierung von auf Oxiranacrylperlen immobilisiertem Trypsin zum Erhalt von Phosphopeptiden aus Casein. Eckstein und Renner (Chemical Abstracts, vol. 122, no. 7, 13. Februar 1995) beschreiben die Immobilisierung von Papain und Trypsin auf Expoxypolymeren für enzymkatalysierte Synthesen. Im US-Patent Nr. 4,994,388 werden mit Collagen beschichtete Polystyrol Mikroträgerpartikel beschrieben. Könnecke et al. (Monatshefte für Chemie, vol. 114, 1983, S. 433-444) beschreiben Peptidsynthesen mit immobilisierten Enzymen. Jakubke et al. (Analytical Chemistry Symposia Series, vol. 9, 1982, S. 529-536) beschreiben die Anwendung von immobilisierten Proteasen zur Peptidsynthese. Könnecke et al. (Monatshefte für Chemie, vol. 112, 1981, S. 469-481) beschreiben die Peptidsynthese durch immobilisierte Enzyme.

Aufgabe der vorliegenden Erfindung ist es, ein Verfahren zur Gewinnung von Insulinen oder deren Analoga aus den entsprechenden Vorläufern, mittels an einem polymeren Träger gebundener Enzyme bereitzustellen, bei dem die unerwünschten Folge- oder Nebenreaktionen weitestgehend vermieden werden.

Die Aufgabe wird gelöst durch Verfahren zur Gewinnung von Insulinen oder deren Analoga aus den entsprechenden Vorläufern, mittels an einem polymeren Träger gebundener Enzyme, das sich dadurch auszeichnet, dass das polymere Trägermaterial keine Poren aufweist.

Das erfindungsgemäße Verfahren ist vorzugsweise zur Gewinnung von Insulinen oder deren Analoga aus den entsprechenden Vorläufern, insbesondere den Präproinsulinen, mittels eines oder mehrerer an einem polymeren Träger gebundener Enzyme geeignet.

Insulinanaloga leiten sich von natürlich vorkommenden Insulinen, nämlich Humaninsulin oder tierischen Insulinen, beispielsweise Schweine- oder Rinderinsulin, durch Substitution oder Fehlen wenigstens eines natürlich auftretenden Aminosäurerestes und/oder Addition wenigstens eines Aminosäurerestes an A- und/oder B-Kette des natürlich vorkommenden Insulins ab.

Vorzugsweise ist das polymere Trägermaterial ein Copolymerisat aus den Monomeren Methacrylamid und N,N'-bis(methacrylamid), wobei das polymere Trägermaterial besonders bevorzugt über oxirangruppenhaltige Monomere verfust.

Bei dem erfindungsgemäßen Verfahren ist das Enzym vorzugsweise mit Hilfe von Oxirangruppen kovalent an das Trägermaterial gebunden.

Bei dem bevorzugten Verfahren zur Gewinnung von Insulinen oder deren Analoga aus den entsprechenden Vorläufern, insbesondere den Präproinsulinen, wird als Enzym vorzugsweise Trypsin verwendet.

Dabei weist das an dem Träger immobilisierte Enzym vorzugsweise eine Aktivität von 0,05 bis 0,5 U/ml auf, der pH-Wert der Reaktionslösung beträgt vorzugsweise 6 bis 10, besonders bevorzugt 7 bis 9.

Im folgenden wird die vorliegende Erfindung insbesondere anhand der Beispiele näher erläutert.

In einer Reihe von Immobilisierungen wurde Trypsin an unterschiedlichen Trägermaterialien kovalent gebunden (Eupergit® C, Eupergit® 250L, Deloxan®).

Der Einsatz dieser Immobilisate bei der Spaltung von Präproinsulin (PPI) lieferte jedoch trotz Variation verschiedener Reaktionsparameter wie z. B. pH-Wert, Temperatur oder Enzymkonzentration die gewünschten Wertstoffe di- bzw. mono-Arg in nur geringer Menge. Sattdessen bildeten sich überwiegend unerwünschte Nebenprodukte wie z.B. des-Thr.

Beim Einsatz von Trypsin, das auf dem Träger Eupergit® C1Z, einem porenfreien Träger, immobilisiert wurde, gelang überraschenderweie die PPI-Spaltung nach dem wie beim nativen Einsatz gewünschten Muster.

Die Wahl von eines porenfreien Trägers, beispielsweise Eupergit® C1Z, bei der Immobilisierung von Trypsin erlaubt somit erstmalig den wiederholten Einsatz des Enzyms mit gleichzeitig sehr guter Selektivität bei der PPI-Spaltung zu Gunsten der beiden Wertstoffe di- und mono-Arg. Eine dem semisynthetischen Verfahren analoge Verwendung des immobilisierten Trypsins mit der Konsequenz einer leichten Abtrennbarkeit des Katalysators von der Reaktionslösung sowie eines mehrfachen Einsatzes ist damit für das gentechnische Verfahren zur Herstellung von Humaninsulin möglich.

### Beispiele

### Beispiel 1 Immobilisierung von Proteinen auf polymeren Trägern

### Beispiel 1.1 Deloxan®

Die Immobilisierung von Proteinen auf Deloxan® setzt eine vorherige Aktivierung des Trägers mit Glutardialdehyd voraus. Bei der eigentlichen Fixierung des Enzyms wurde in einer Meßreihe die aufgegebene native Aktivität des Trypsins variiert.

| | | |
|---|---|---|
| Aktivierung: pH = | 9 | [KPP: 100 mM] |
| T = | 25 °C | |
| t = | 1 h | |
| Deloxan® = | 10 g | [VR = 150 ml] |
| GD = | 0,2 g/gTM | |

| | | |
|---|---|---|
| Fixierbedingungen: pH = | 9 | [KPP: 100 mM] |
| T = | 25 °C | |
| t = | 3 h | |

### Beispiel 1.2 Eupergit® C250L und Eupergit® C

Bei der Immobilisierung von Trypsin auf Eupergit®-Trägern wird in einer einstufigen Reaktion die Trägersuspension mit der Enzymlösung versetzt.

| Fixierbedingungen für Eupergit® C250L: | | |
|---|---|---|
| pH = | 8 | [KPP: 1 M] |
| T = | 25 °C | |
| t = | 3 d | |

| Fixierbedingungen für Eupergit® C: | | |
|---|---|---|
| pH = | 8,5 | [Boratpuffer 100 mM] |
| T = | 25 °C | |
| t = | 3 d | |
| Benzamidin = | 24 mM | |

### Beispiel 1.3 Eupergit® C1Z

Der Träger Eupergit® C1Z zeichnet sich im Gegensatz zu den übrigen Trägern durch Porenfreiheit aus.

| Fixierbedingungen: | | |
|---|---|---|
| pH = | 8 | [KPP: 1 M] |
| T = | 25 °C | |
| t = | 3 d | |
| nat. Trypsin = | 7.400 U/gTM | [Σ =37.000 U auf 5 g] |

Unter den gegebenen Bedingungen wird eine spez. Aktivität von 405 U/gTM erzielt. Während die Fixierausbeute mit den Ergebnissen der beiden anderen Eupergit®-Träger vergleichbar ist, liegt die Wiederfindungsrate mit 46 % deutlich höher.

### Beispiel 2

Dieses Beispiel dient Vergleichszwecken mit dem beanspruchten Verfahren.

In einem Becherglas wird ein Volumen von 1 I der Präproinsulin-Lösung mit einer Konzentration von 0,83 mg/ml vorgelegt. Nach Einstellung von pH 8,3 wird die Spaltreaktion durch Zugabe des auf Deloxan® fixierten Trypsins in einer Konzentration von 0,81 U/ml gestartet. Über den gesamten Reaktionszeitraum beträgt die Temperatur der Lösung 6 °C. Der pH-Wert wird während der Umsetzung durch Zugabe von 1 M NaOH-Lösung konstant gehalten. In regelmäßigen Abständen werden über einen Zeitraum von 23 h Proben der Reaktionslösung gezogen und der Gehalt der einzelnen Reaktionskomponenten mittels HPLC bestimmt.

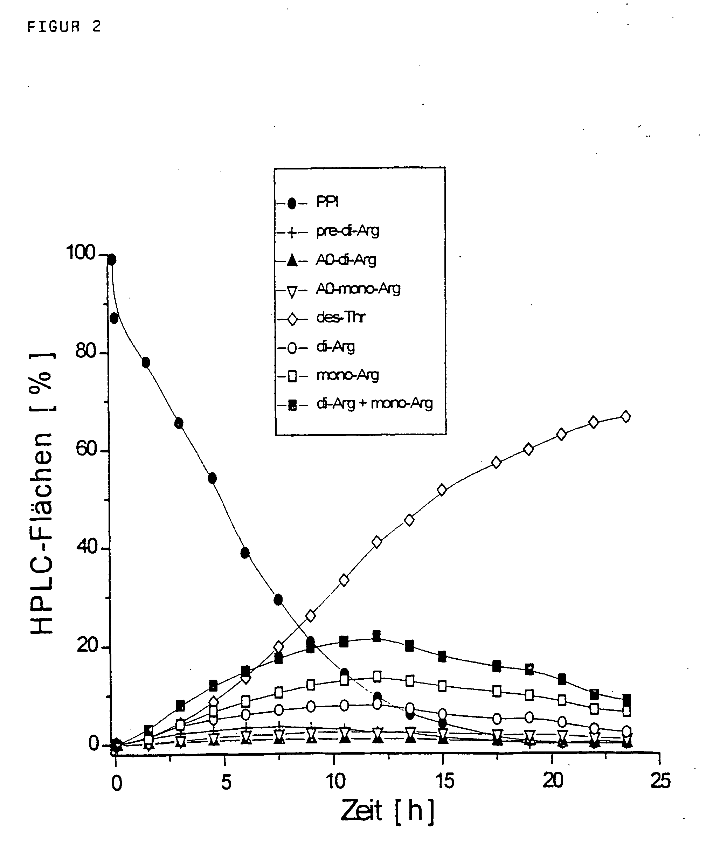

Wie in Fig. 2 zu sehen, entsteht über den betrachteten Zeitraum des-Thr als Hauptprodukt, während die Menge der beiden Wertstoffe di- und mono-Arg deutlich niedriger liegt.

### Beispiel 3

Dieses Beispiel dient Vergleichszwecken mit dem beanspruchten Verfahren.

In einem Becherglas wird ein Volumen von 1 I der Präproinsulin-Lösung mit einer Konzentration von 0,83 mg/ml vorgelegt. Nach Einstellung von pH 8,3 wird die Spaltreaktion durch Zugabe des auf Eupergit® C250L fixierten Trypsins in einer Konzentration von 1,62 U/ml gestartet. Über den gesamten Reaktionszeitraum beträgt die Temperatur der Lösung 6 °C. Der pH-Wert wird während der Umsetzung durch Zugabe von 1 M NaOH-Lösung konstant gehalten. In regelmäßigen Abständen werden über einen Zeitraum von 23 h Proben der Reaktionslösung gezogen und der Gehalt der einzelnen Reaktionskomponeneten mittels HPLC bestimmt.

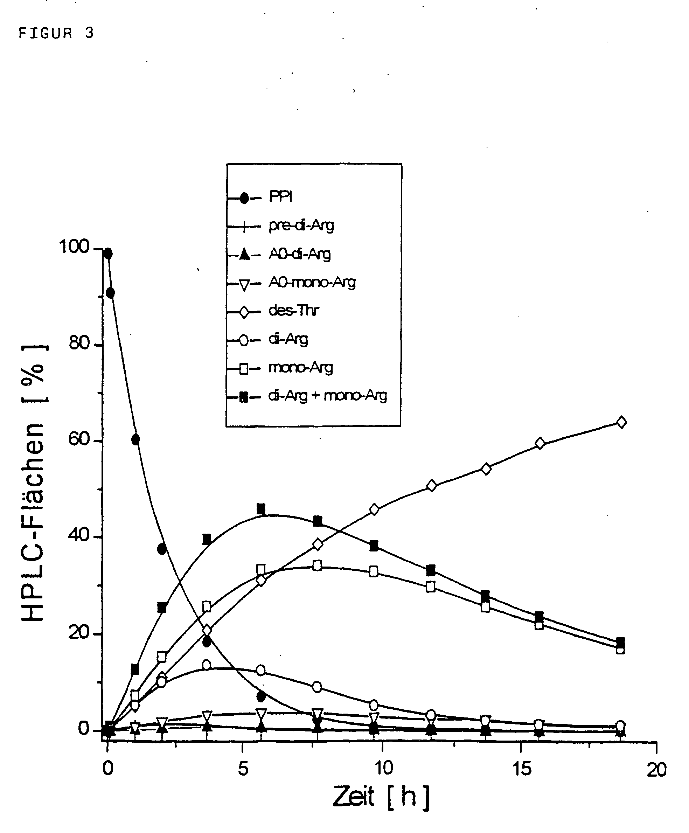

Wie in Fig. 3 zu sehen, entsteht auch in diesem Fall über den betrachteten Zeitraum des-Thr als Hauptprodukt, während die Menge der beiden Wertstoffe di- und mono-Arg, die darüberhinaus in einer Folgereaktion wieder abgebaut werden, deutlich niedriger liegt.

### Beispiel 4

In einem Becherglas wird ein Volumen von 1 I der Präproinsulin-Lösung mit einer Konzentration von 0,83 mg/ml vorgelegt. Nach Einstellung von pH 8,3 wird die Spaltreaktion durch Zugabe des auf Eupergit® C1Z fixierten Trypsins in einer Konzentration von 0,081 U/ml gestartet. Über den gesamten Reaktionszeitraum beträgt die Temperatur der Lösung 6 °C. Der pH-Wert wird während der Umsetzung durch Zugabe von 1 M NaOH-Lösung konstant gehalten. In regelmäßigen Abständen werden über einen Zeitraum von 23 h Proben der Reaktionslösung gezogen und der Gehalt der einzelnen Reaktionskomponeneten mittels HPLC bestimmt.

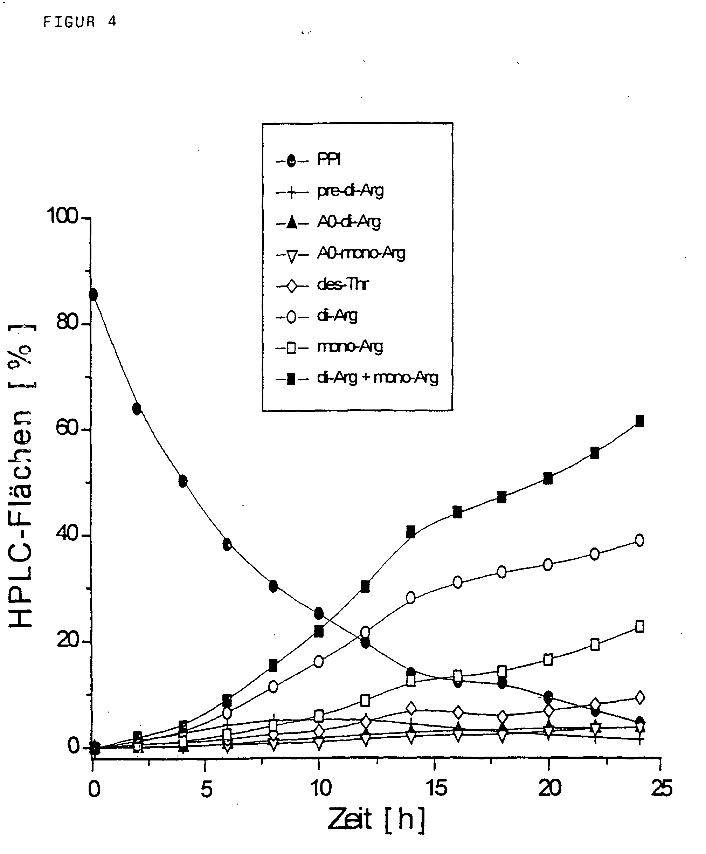

Wie in Fig. 4 zu sehen, entstehen im Gegensatz zu den vorherigen Umsetzungsversuchen mit immobilisiertem Trypsin auf der Grundlage dieses Trägers die beiden gewünschten Wertstoffe di- und mono-Arg im Überschuß, während der Anteil der Nebenprodukte (insbesondere des-THr) drastisch herabgesetzt wird.

### Beispiel 5

In einem Becherglas wird ein Volumen von 1 l der Präproinsulin-Lösung mit einer Konzentration von 0,83 mg/ml vorgelegt. Nach Einstellung von pH 8,3 wird die Spaltreaktion durch Zugabe des auf Eupergit® C1Z fixierten Trypsins in einer Konzentration von 0,405 U/ml gestartet. Über den gesamten Reaktionszeitraum beträgt die Temperatur der Lösung 6 °C. Der pH-Wert wird während der Umsetzung durch Zugabe von 1 M NaOH-Lösung konstant gehalten. In regelmäßigen Abständen werden über einen Zeitraum von 12 h Proben der Reaktionslösung gezogen und der Gehalt der einzelnen Reaktionskomponeneten mittels HPLC bestimmt. Auch in diesem Beispiel entsteht beim Einsatz des auf Eupergit® C1Z immobilisierten Trypsins das gewünschte Produkt- und Nebenprodukt-spektrum.

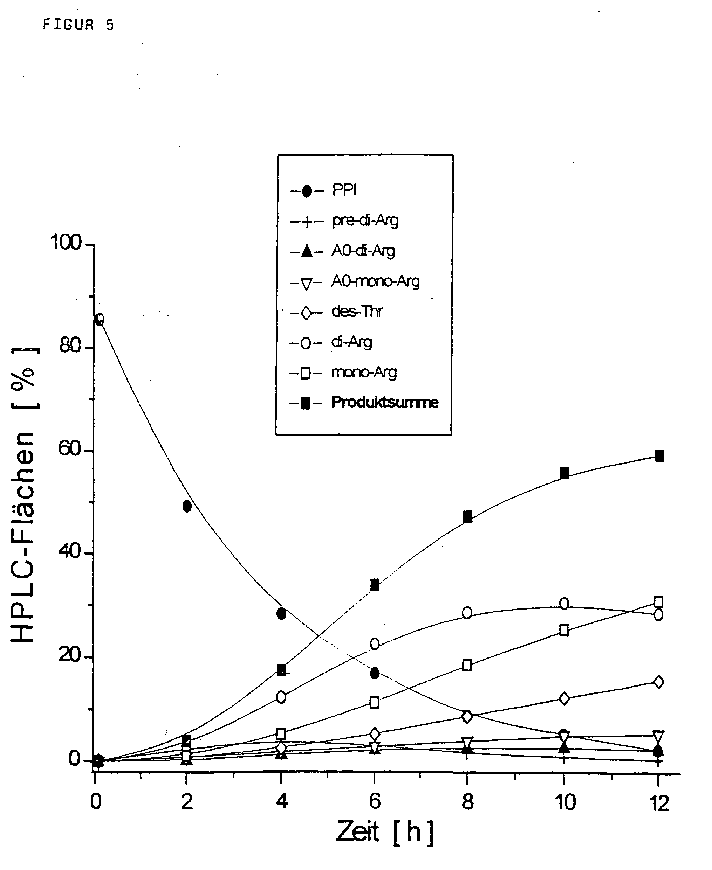

Wie in Fig. 5 zu sehen, kann durch die Verfünfachung der Katalysatorkonzentration die Umsetzungsgeschwindigkeit erhöht und damit die benötigte Reaktionszeit deutlich verkürzt werden, ohne daß sich dadurch die Selektivität der Reaktion zu Ungunsten der beiden Wertstoffe verschiebt.

### Mögliche Deutung der Ergebnisse der Beispiele 2 bis 5

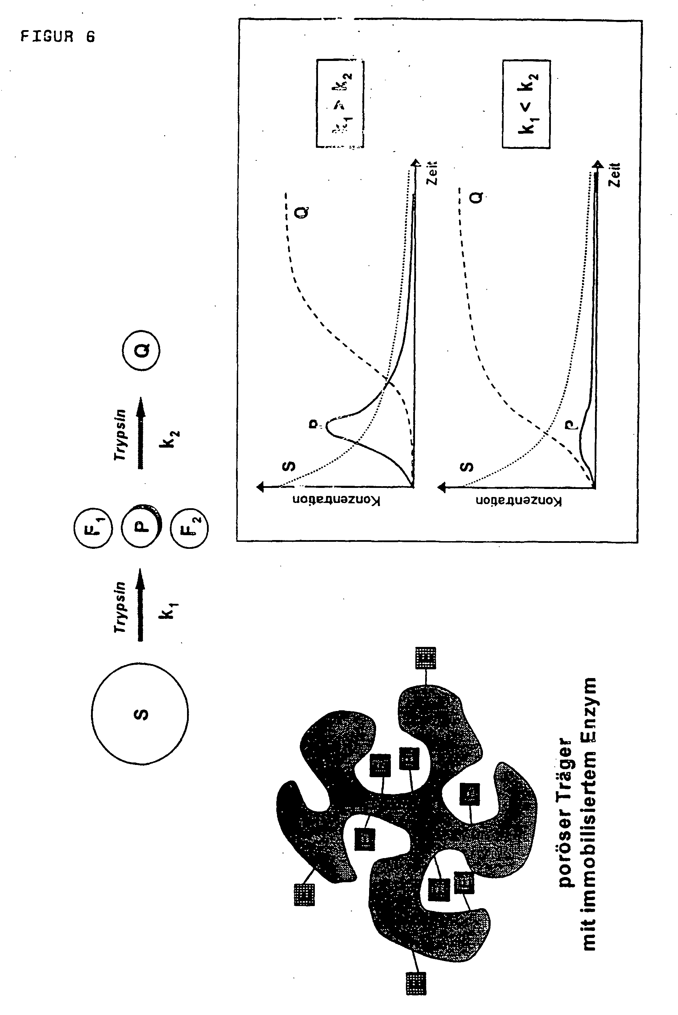

Beispielsweise im Falle von Präproinsulinen (PPI) handelt es sich bei dem Ausgangssubstrat im Gegensatz zu den Folgeprodukten der Reaktion um eine größeres Molekül (≅ 10 kDa), das einer stärkeren Porendiffusionslimitierung unterliegt. Die Reaktionsgeschwindigkeit aller Folgereaktionen ist aus diesem Grunde größer als die direkte Umsetzung von PPI in unterschiedliche Fragmente. Bei porenbehafteten Trägern sollte aus diesem Grunde eine Akkumulation des gewünschten Zwischenproduktes P (s. Fig. 6; in diesem Bsp.: di-Arg) nicht möglich sein. Als Lösungsansatz zur Steuerung der Selektivität bietet sich daher der Einsatz porenfreier (bzw. nahezu porenfreier) Träger an.

### Figurenlegende:

- Fig. 1:: Reaktive Stellen bei der tryptischen Spaltung von Präproinsulin (INS = Insulin; AO-Arg-INS = Arg(A0)-Insulin; INS-di-Arg = Arg(B31),Arg(B32)-Insulin; INS-mono-Arg = Arg(B31)-Insulin; des-Thr = Des(B30)-Insulin).
- Fig. 2:: Verlauf der Konzentrationen der Reaktionskomponenten als Funktion der Zeit bei der Spaltung von Preproinsulin mit auf Deloxan® immobilisiertem Trypsin.
- Fig. 3:: Verlauf der Konzentrationen der Reaktionskomponenten als Funktion der Zeit bei der Spaltung von Preproinsulin mit auf Eupergit® C250L immobilisiertem Trypsin.
- Fig. 4:: Verlauf der Konzentrationen der Reaktionskomponenten als Funktion der Zeit bei der Spaltung von Preproinsulin mit auf Eupergit® C1Z immobilisiertem Trypsin.
- Fig. 5:: Verlauf der Konzentrationen der Reaktionskomponenten als Funktion der Zeit bei der Spaltung von Preproinsulin mit auf Eupergit® C1Z immobilisiertem Trypsin.
- Fig. 6:: Bedeutung der Trägermorphologie für die Selektivität der Reaktion (S = Substrat, z.B. PPI; P = gewünschtes Zwischenprodukt, z.B. Di-Arg; Q = unerwünschtes Folgeprodukt, z.B. des-Thr).

## Patentansprüche

1. Verfahren zur Gewinnung von Insulinen oder deren Analoga aus den entsprechenden Vorläufern mittels eines oder mehrerer an einem polymeren Träger gebundener Enzyme, **dadurch gekennzeichnet, daß** das polymere Trägermaterial keine Poren aufweist.

2. Verfahren nach Anspruch 1, **dadurch gekennzeichnet, daß** das polymere Trägermaterial ein Copolymerisat aus den Monomeren Methacrylamid und N,N'-bis(methacrylamid) ist.

3. Verfahren nach einem oder mehreren der Ansprüche 1 oder 2, **dadurch gekennzeichnet, daß** das polymere Trägermaterial über oxirangruppenhaltige Monomere verfügt.

4. Verfahren nach einem oder mehreren der Ansprüche 1 bis 3, **dadurch gekennzeichnet, daß** das Enzym mit Hilfe von Oxirangruppen kovalent an das Trägermaterial gebunden ist.

5. Verfahren nach einem oder mehreren der Ansprüche 1 bis 4, **dadurch gekennzeichnet, daß** das Enzym Trypsin ist.

6. Verfahren nach einem oder mehreren der Ansprüche 1 bis 5, **dadurch gekennzeichnet, daß** das an dem Träger immobilisierte Enzym eine Aktivität von 0,05 bis 0,5 U/ml aufweist.

7. Verfahren nach einem oder mehreren der Ansprüche 1 bis 6, **dadurch gekennzeichnet, daß** der pH-Wert der Reaktionslösung 6 bis 10 beträgt.

8. Verfahren nach Anspruch 7, **dadurch gekennzeichnet, daß** der pH-Wert 7 bis 9 beträgt.

## Claims

1. A process for the obtainment of insulins or their analogs from the corresponding precursors by means of one or more enzymes bonded to a polymeric support, wherein the polymeric support material has no pores.

2. The process as claimed in claim 1, wherein the polymeric support material is a copolymer of the monomers methacrylamide and N,N'-bis(methacrylamide).

3. The process as claimed in one or more of claims 1 and 2, wherein the polymeric support material has oxirane group-containing monomers.

4. The process as claimed in one or more of claims 1 to 3, wherein the enzyme is bonded covalently to the support material with the aid of oxirane groups.

5. The process as claimed in one or more of claims 1 to 4, wherein the enzyme is trypsin.

6. The process as claimed in one or more of claims 1 to 5, wherein the enzyme immobilized on the support has an activity of 0.05 to 0.5 U/ml.

7. The process as claimed in one or more of claims 1 to 6, wherein the pH of the reaction solution is 6 to 10.

8. The process as claimed in claim 7, wherein the pH is 7 to 9.

## Revendications

1. Procédé pour l'obtention d'insulines ou de leurs analogues à partir des précurseurs correspondants, au moyen d'une ou plusieurs enzymes fixées sur un support polymère, **caractérisé en ce que** la matière de support polymère ne présente pas de pores.

2. Procédé selon la revendication 1, **caractérisé en ce que** la matière de support polymère est un copolymère formé à partir des monomères méthacrylamide et N,N'-bis(méthacrylamide).

3. Procédé selon une ou plusieurs des revendications 1 et 2, **caractérisé en ce que** la matière de support polymère est dotée de monomères contenant des groupes oxiranne.

4. Procédé selon une ou plusieurs des revendications 1 à 3, **caractérisé en ce que** l'enzyme est fixée à la matière de support par liaison covalente à l'aide de groupes oxiranne.

5. Procédé selon une ou plusieurs des revendications 1 à 4, **caractérisé en ce que** l'enzyme est la trypsine.

6. Procédé selon une ou plusieurs des revendications 1 à 5, **caractérisé en ce que** l'enzyme immobilisée sur le support présente une activité de 0,05 à 0,5 U/ml.

7. Procédé selon une ou plusieurs des revendications 1 à 6, **caractérisé en ce que** le pH de la solution réactionnelle va de 6 à 10.

8. Procédé selon la revendication 7, **caractérisé en ce que** le pH va de 7 à 9.
